# EUROPEAN PATENT APPLICATION

(11) **EP 3 031 421 A1**
(43) Date of publication of application: **15.06.2016**
(21) Application number: 15195937.6
(22) Date of filing: 23.11.2015
(51) Int. Cl.: A61B 18/14, A61B 18/00

(54) **ELECTRODE ASSEMBLY**

(30) Priority: 11.12.2014 GB 201422059
(71) Applicant: Gyrus Medical Limited, Cardiff CF3 0LT (GB)
(72) Inventor: Benn, Christopher Charles, Bristol, BS35 5RE (GB)
(74) Representative: Wallin, Nicholas James

(57) **Abstract**

An electrode assembly for an electrosurgical instrument comprises an electrode tip (4) mounted on an elongate electrode stem (5), and an insulating housing (3), the housing having an elongate bore (8) formed therethrough. The bore (8) is such that the electrode stem (5) can be received within the bore with the electrode tip (4) extending from the distal end of the housing and a proximal portion (9) of the stem extending from the proximal end of the housing. An electrical lead (10) is located adjacent the proximal portion (9) of the stem, and a mass (11) of electrically conductive material forms an electrical connection between the lead (10) and the electrode stem (5). The mass (11) of electrically conductive material is of such a size that in addition to forming an electrical connection it also forms a mechanical stop preventing the electrode stem (5) from being moved distally with respect to the insulating housing.

## Description

### Technical Field

Embodiments of the invention relate to an electrode assembly for an electrosurgical instrument suitable for the treatment of tissue. Such instruments are commonly used for the vaporisation and/or coagulation of tissue in surgical intervention, most commonly in "keyhole" or minimally invasive surgery, but also in "open" surgery.

### Background to the Invention and Prior Art

Electrosurgical instruments are growing in sophistication and complexity, with the number of electrodes increasing and the size of electrodes decreasing. One of the major factors in electrosurgical instrument design is the difficulty and complexity of instrument assembly, as well as the increased risk of components breaking or becoming detached during use. However, there is still a need for simple and efficient electrosurgical instruments provided that all safety and instrument reliability requirements are met.

### Summary of the Invention

Embodiments of the present invention attempt to address the above problem by providing a simple and efficient electrosurgical instrument, while still providing acceptable safety and reliability standards.

Accordingly, an electrode assembly for an electrosurgical instrument is provided by one embodiment, the electrode assembly including an electrode comprising an electrode tip mounted on an elongate electrode stem, an insulating housing having a proximal end and a distal end, the housing also having an elongate bore formed therethrough, such that the electrode stem can be received within the bore with the electrode tip extending from the distal end of the housing and a proximal portion of the stem extending from or adjacent to the proximal end of the housing, an electrical lead located adjacent the proximal portion of the stem, and a mass of electrically conductive material, the mass of electrically conductive material forming an electrical connection between the lead and the electrode stem, characterised in that the mass of electrically conductive material is of such a size that in addition to forming an electrical connection it also forms a mechanical stop preventing the electrode stem from being moved distally with respect to the insulating housing.

The mass of electrically conductive material acts, in addition to making an electrical connection between the lead and the electrode, as a retention mechanism preventing the electrode stem from moving distally with respect to the bore in the insulating housing. Whereas the mass of electrically conductive material is typically capable, by itself, of preventing the electrode stem from being moved forwardly with respect to the insulating housing, there may additionally be provided a further retention mechanism, such as an adhesive, locking member or other retention means. Typically, the mass of electrically conductive material acts as a secondary retention mechanism, with the further retention mechanism providing the primary means of retention of the electrode with respect to the insulating housing. However, even if complemented by a further retention mechanism, the mass of electrically conductive material acts as a backup to retain the electrode in the insulating housing even if there should be a failure of the further (primary) retention mechanism. Furthermore, the mass of electrically conductive material acts to secure the electrode in place with respect to the insulating housing during assembly, and before the further retention mechanism has been applied.

Conveniently, the insulating housing has a shoulder associated with the proximal end thereof, the mass of electrically conductive material engaging the shoulder to prevent the electrode stem moving forwardly with respect to the housing. The shoulder is typically formed by a portion of the proximal end face of the housing.

According to one convenient arrangement, the mass of electrically conductive material is solder. Rather than merely connecting the lead to the electrode with the minimum amount of solder necessary to form an electrical connection, a significant mass of solder is used, such that its diameter is greater than that of the bore in the insulating housing, thereby ensuring that it also acts as a mechanical stop to retain the electrode in the housing.

Alternatively, the mass of electrically conductive material is conceivably a copper alloy used in a braising operation. Whether the flowable material is used in a soldering or braising operation, the amount used is sufficient for the conductive material to form a mechanical stop, in addition to an electrical connection.

According to a further convenient arrangement, the mass of electrically conductive material is conceivably a mass of the lead and/or the electrode stem, formed in a welding operation. In this arrangement, the lead is welded to the electrode stem, but in a way such that a mass of material of a diameter larger than that of the electrode stem is created at the join. In this way, the weld acts as a mechanical stop to retain the electrode within the insulating housing, in addition to forming an electrical connection between the lead and the electrode.

Finally, according to a further alternative arrangement, the mass of electrically conductive material is conceivably an electrically conductive adhesive. Such adhesives are widely available, and provide not only an electrical connection between the lead and its associated electrode, but also a mass of material capable of acting as a mechanical stop to prevent the electrode stem from passing through the bore in the insulating housing. Whichever method of attachment is employed, the mass of electrically conductive material acts both as an electrical connection and also as a mechanical stop to retain the electrode within the housing.

Embodiments of the invention further reside in a method of assembling an electrode assembly for an electrosurgical instrument, including the steps of
a) forming an electrode with an electrode tip mounted on an elongate electrode stem,
b) forming an insulating housing with a proximal end and a distal end, and having an elongate bore formed therethrough,
c) presenting the electrode to the housing such that the electrode is temporarily located in the housing, with the electrode stem received within the bore and with the electrode tip extending from the distal end of the housing and a proximal portion of the stem extending from or adjacent to the proximal end of the housing,
d) presenting an electrical lead to the proximal portion of the electrode stem, and
e) connecting the lead to the proximal portion of the stem in such a way as to create a mass of electrically conductive material attached to the electrode stem so as to form not only an electrical connection between the lead and the stem, but also a mechanical stop preventing the electrode stem from being moved forwardly with respect to the insulating housing.

Conveniently, the step of connecting the lead to the electrode stem is by soldering, and the mass of electrically conductive material is solder. Alternatively, the step of connecting the lead to the electrode stem is by braising, and the mass of electrically conductive material is a copper alloy. Alternatively, the step of connecting the lead to the electrode stem is by welding, and the mass of electrically conductive material is a part of the electrode stem melted by the welding process. Alternatively, the step of connecting the lead to the electrode stem is by using an adhesive, and the mass of electrically conductive material is an electrically conductive adhesive. Alternatively the method includes the further step of subsequently applying an adhesive between the electrode and the insulating housing to form an additional retention mechanism.

### Description of the Drawings

Embodiments of the invention will now be further described, by way of example only, with reference to the accompanying drawings, in which:
Figure 1 is a perspective view of an electrode assembly in accordance with an embodiment of the present invention,
Figure 2 is a schematic sectional view of a the electrode assembly of Figure 1, and
Figure 3 is a schematic sectional view of an alternative embodiment of electrode assembly in accordance with the present invention.

### Description of the Embodiments

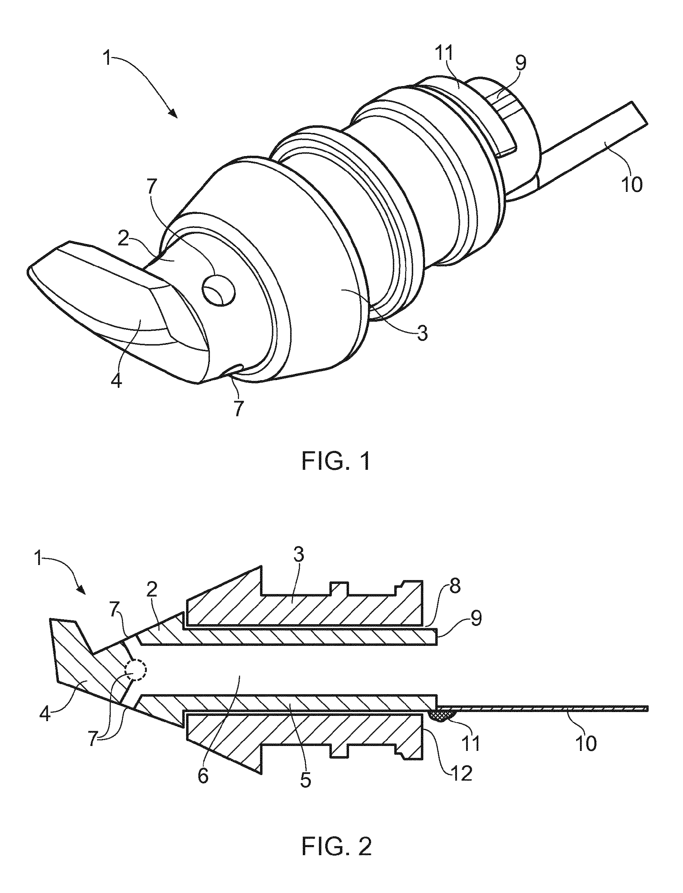

Referring to Figures 1 & 2, an electrode assembly for an electrosurgical instrument is shown generally at 1, and comprises a chisel tip electrode 2 and an insulating housing 3. The electrode 2 comprises an electrode tip 4 and an elongate stem 5, the electrode having a suction lumen 6 therein leading to a plurality of suction apertures 7 in the electrode tip 4.

The insulating housing contains a central bore 8 into which the stem 5 of the electrode 2 can be received. During assembly of the electrosurgical instrument, the electrode 2 is introduced into the housing 3, with the stem 5 of the electrode being received within the bore 8 of the housing. The stem 5 is slightly longer than the bore of the housing 3, such that a proximal end 9 of the stem protrudes from the housing. In other embodiments, however, the stem 5 may be substantially the same length as the bore of the housing such that the proximal end 9 of the stem is adjacent to the end of the bore of the housing 3. In either case, a metallic lead 10 is placed adjacent the proximal end 9 of the stem, and is attached thereto by means of a mass 11 of electrically conductive flowable material such as solder.

The mass of solder 11 is of such a size that if an attempt is made to move the electrode 2 in a distal direction the mass 11 will come into contact with a shoulder 12 formed by the proximal end of the housing 3. Thus the mass 11 not only acts to form an electrical connection between the lead 10 and the electrode 2, but it also acts as a mechanical stop to locate the electrode 2 within the housing 3. The mass may act as the sole mechanism for retaining the electrode within the housing, but more normally the mass acts as a secondary retention mechanism, with an additional means such as an adhesive (not shown) being applied to act as the primary retention mechanism. However, the secondary retention mechanism provided by the mass 11 has several advantages. Firstly it acts as an initial retention means, to locate the electrode 2 within the housing 3 during assembly until the adhesive or other primary retention means can be applied. Secondly, should the primary retention mechanism fail during use of the instrument, the mass 11 ensures that the electrode 2 is still retained within the housing 3 during use, rather than possibly becoming detached during the surgical procedure.

The mass 11 of electrically conductive flowable material may not be solder, but could alternatively be a mass of copper alloy used in a braised connection between the lead 10 and the electrode stem 5. The mass 11 could also be an electrically conductive adhesive, such adhesives being widely available from a range of commercial suppliers.

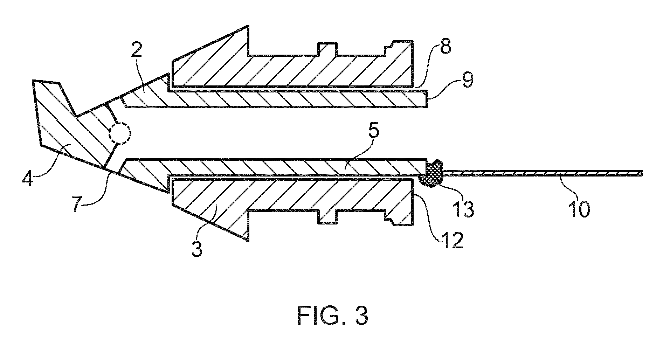

Figure 3 shows a further alternative, in which the lead 10 has been welded to the proximal end 9 of the stem 5 such that a bead 13 of fused material is formed between the lead and the stem 5. The welding operation is conducted such that the bead 13 formed by the fused material is of such a size that it protrudes from the joint so as to abut the shoulder 12 formed by the proximal end of the housing 3 should an attempt be made to move the electrode 2 in a distal direction. Thus, in the same way as the mass of solder or copper alloy described with reference to Figures 1 & 2, the bead 13 of fused material acts as a mechanical stop in addition to forming an electrical connection between the lead 10 and the electrode 2.

Other embodiments will be apparent to those skilled in the art without departing from the scope of the present invention. The electrical connection between the lead and the electrode also forms a mechanical stop to prevent the electrode being removed from the insulating housing in which it is housed.

## Claims

1. An electrode assembly for an electrosurgical instrument, the electrode assembly comprising an electrode tip mounted on an elongate electrode stem, an insulating housing having a proximal end and a distal end, the housing also having an elongate bore formed therethrough, such that the electrode stem can be received within the bore with the electrode tip extending from the distal end of the housing and a proximal portion of the stem extending from or adjacent to the proximal end of the housing, an electrical lead located adjacent the proximal portion of the stem, and a mass of electrically conductive material, the mass of electrically conductive material forming an electrical connection between the lead and the electrode stem, **characterised in that** the mass of electrically conductive material is of such a size that in addition to forming an electrical connection it also forms a mechanical stop preventing the electrode stem from being moved distally with respect to the insulating housing.

2. An electrode assembly according to claim 1, wherein the mass of electrically conductive material is capable, by itself, of preventing the electrode stem from being moved forwardly with respect to the insulating housing.

3. An electrode assembly according to claim 1 or claim 2, wherein the insulating housing has a shoulder associated with the proximal end thereof, the mass of electrically conductive material engaging the shoulder to prevent the electrode stem moving forwardly with respect to the housing.

4. An electrode assembly according to claim 3, wherein the shoulder is formed by a portion of the proximal end face of the housing.

5. An electrode assembly according to any preceding claim, wherein the mass of electrically conductive material is solder.

6. An electrode assembly according to any of claims 1 to 4, wherein the mass of electrically conductive material is a copper alloy used in a braising operation.

7. An electrode assembly according to any of claims 1 to 4, wherein the mass of electrically conductive material is a mass of the lead and/or the electrode stem, formed in a welding operation.

8. An electrode assembly according to any of claims 1 to 4, wherein the mass of electrically conductive material is an electrically conductive adhesive.

9. An electrosurgical instrument including an electrode assembly according to any of claims 1 to 8.

10. A method of assembling an electrode assembly for an electrosurgical instrument including the steps of
a) forming an electrode with an electrode tip mounted on an elongate electrode stem,
b) forming an insulating housing with a proximal end and a distal end, and having an elongate bore formed therethrough,
c) presenting the electrode to the housing such that the electrode is temporarily located in the housing, with the electrode stem received within the bore and with the electrode tip extending from the distal end of the housing and a proximal portion of the stem extending from or adjacent to the proximal end of the housing,
d) presenting an electrical lead to the proximal portion of the electrode stem, and
e) connecting the lead to the proximal portion of the stem in such a way as to create a mass of electrically conductive material attached to the electrode stem so as to form not only an electrical connection between the lead and the stem, but also a mechanical stop preventing the electrode stem from being moved diatally with respect to the insulating housing.

11. A method according to claim 10, wherein the step of connecting the lead to the electrode stem is by soldering, and the mass of electrically conductive material is solder.

12. A method according to claim 10, wherein the step of connecting the lead to the electrode stem is by braising, and the mass of electrically conductive material is a copper alloy.

13. A method according to claim 10, wherein the step of connecting the lead to the electrode stem is by welding, and the mass of electrically conductive material is a part of the electrode stem melted by the welding process.

14. A method according to claim 10, wherein the step of connecting the lead to the electrode stem is by using an adhesive, and the mass of electrically conductive material is an electrically conductive adhesive.

15. A method according to any of claims 10 to 14, including the further step of subsequently applying an adhesive between the electrode and the insulating housing to form an additional retention mechanism.
